# EUROPEAN PATENT SPECIFICATION

(11) **EP 2 549 262 B1**
(45) Date of publication and mention of the grant of the patent: **06.01.2016**
(21) Application number: 12158924.6
(22) Date of filing: 09.03.2012
(51) Int. Cl.: G01N 3/34, G01N 3/00, G01N 33/00

(54) **Machine to perform tests on materials**
Machine zur Materialprüfung
Machines d'essai des matériaux

(30) Priority: 22.07.2011 IT VR20110156
(43) Date of publication of application: 23.01.2013
(73) Proprietor: Vibram S.p.A., 21041 Albizzate (Varese) (IT)
(72) Inventor: Bramani, Marco, 21041 Albizzate (Varese) (IT); Gabbrielli, Giorgio, 21041 Albizzate (Varese) (IT); Sacchi, Massimiliano, 21041 Albizzate (Varese) (IT)
(74) Representative: Feltrinelli, Secondo Andrea

(56) References cited:
- GB-A- 376 726
- JP-A- 2003 227 785
- JP-U- 62 121 544
- US-A1- 2006 005 606
- US-A1- 2011 016 983

## Description

### TECHNICAL FIELD OF THE INVENTION

The present invention refers to a machine for carrying out tests on materials, in particular on materials used in the footwear industry for making for example soles, midsoles and insoles.

### STATE OF THE PRIOR ART

Machines for carrying out fatigue tests on various materials are currently known.

The most common embodiments of machines for carrying out fatigue tests include dynamometers which allow applying numerous tensile stress cycles and subsequent compression to a suitably shaped test piece. The applied stress is managed through a closed loop control cycle. However, this embodiment does not allow applying a stress of the pulse type, capable of simulating for example an impact, to the material to be tested. Thus, it is not possible to determine the toughness of the material, i.e. the capacity to absorb energy, for example following an impact, and use it for the deformation thereof.

Machines capable of allowing carrying out impact tests on materials, so as to be able to determine the toughness thereof, are also known.

The most common embodiment consists in an instrumented pendulum, also known as Charpy pendulum, which mainly comprises a rod, a drop hammer and a suitably shaped test piece.

An end of the rod is fastened through a hinge to the machinery, while at the other end of the rod a drop hammer is connected which impacts against the test piece. Though this machine allows determining the toughness of the material it however does not allow simulating a cyclic pulse load. The stress due to a series of repeated impacts over time, generally corresponds to the condition to which the footwear soles, midsoles and insoles are subjected during use thereof. Furthermore, this machine does not allow determining the deformation value of the test pieces following the impact. Actually, normally the test piece is broken by the drop hammer during the test. <...>

### OBJECTS OF THE INVENTION

An object of the present invention is to propose a machine capable of allowing applying a compression load of the pulse type, which simulates an impact, to the test material.

Furthermore such machine should allow applying the load also cyclically, allowing to carry out fatigue tests on different materials, in particular on materials used in the footwear industry, for making <Patent application JP 2003-227785 A discloses an apparatus to repeatively apply a fixed load to a sheet sensor. The sheet sensor is foreseen to be provided inside the seats of a vehicles in order to verify the presence of a passenger on the seat. The apparatus comprises a frame, a drive motor able to move a disc to which a weight is connected by a kinematic motion, and a placement stand above which the sheet sensor to be tested is placed. Such kinematic motion is of the conrod-crank type.

Patent application US 2011/0016983 A1 discloses a testing device for the cyclically application of a static or dynamic torsion to a sample. The testing device comprises a first device, for clamping one end of the sample, and a second device for clamping the other end of the sample. The second clamping device is connected to a drive motor for the movement of the sample respect to the first clamping device.

The testing device comprises a force transducer suitable to measure the force applied to the sample.

Utility model JP 62121544 U discloses a device for the application of an impulsive load to a sample material, comprising a basement, above which the material sample is placed, a mobile rod provided at one end of a knocker mass, means for guiding the mobile rod respect to the basement, and drive motor means for the cyclic movement of the rod.

The rod is connected to the basement, at the opposite side of the guiding means, through a return spring.>

for example soles, midsoles and insoles through a solution that is rational, easy, efficient to use and inexpensive.

According to an aspect of the present invention a machine according to claim 1 is provided.

The dependent claims refer to preferred and advantageous embodiments of the invention.

### BRIEF DESCRIPTION OF THE DRAWINGS

Further characteristics and advantages of the present invention will be clearer from the detailed description of a preferred but non-exclusive embodiment of a machine for applying a load of the pulse and cyclic type to a test material, provided by way of non-limiting example, in the attached drawings wherein:
figure 1 is a schematic view of the machine;
figure 2 is a schematic view of the machine;
figure 3 is a schematic view of a further possible embodiment of the machine;
figure 4 is a diagram regarding figures 1, 2;
figure 5 is a diagram regarding figure 3.

### EMBODIMENTS OF THE INVENTION

With reference to the attached figures, a machine for carrying out tests on materials is schematically indicated with 1. Such machine 1 allows applying a compression load of the pulse type, simulating an impact, to a material.

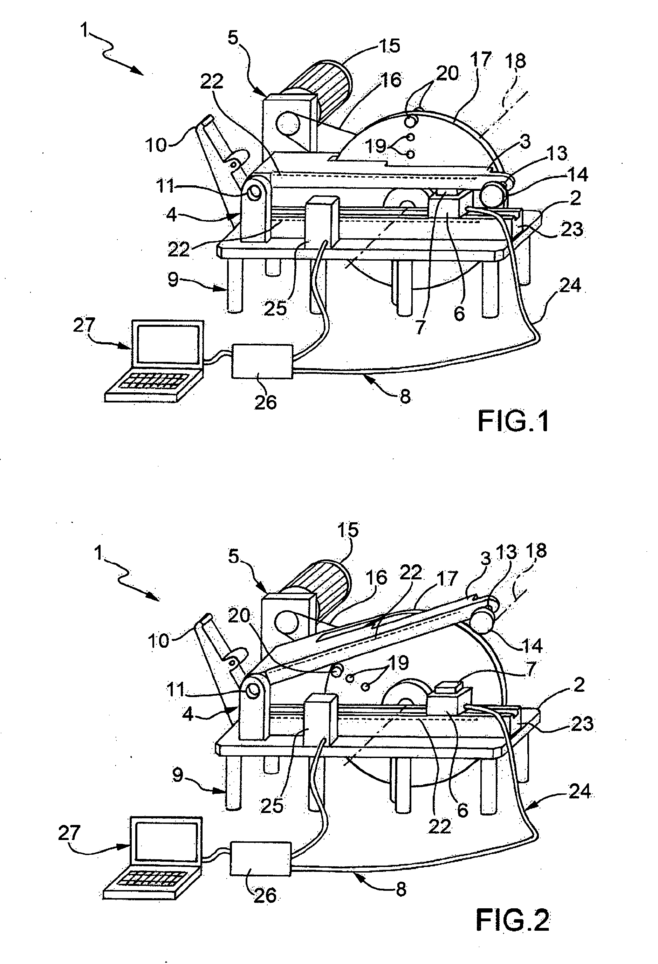

The machine 1 further allows cyclically applying such load, simulating the stresses that the materials used in the footwear industry for making, particularly, soles, midsoles and insoles, are subjected to. Furthermore, the machine 1 allows extrapolating the stress/deformation constitutive bond curve of the material detecting the intensity of the stress and the deformation the test material is subjected to. With reference to the embodiment of figures 1 and 2, the machine 1 mainly comprises a basement 2, a movable rod 3, means 4 for guiding the rod 3 with respect to the basement 2, a device 5 for moving the rod 3, a support 6 constrained to the basement 2, on which the material to be tested 7 is placed, and an electronic device 8 for extrapolating the data regarding the stress and deformation that the test material 7 is subjected to.

The basement 2 is provided with a series of feet 9 for allowing the basement 2 to rest on the ground. Furthermore, the basement 2 is provided with an element 10 for supporting the rod 3 when the machine 1 is not operating and allow safely positioning the support 6 on the basement 2 and the test material 7 on the support 6.

The guiding means 4, obtained for example by means of a hinge 11, allow a relative rotation of the rod 3 with respect to the basement 2.

The rod 3 is provided with a suitable support 13 for connecting a mass 14 to the rod 3. Modifying the mass 14 allows varying the force with which the rod 3 impacts against the test material 7, stressing the material with impacts of various degrees and simulating, for example, the stress to which the materials used for making soles, midsoles and insoles are usually subjected to when walking or running.

The device 5 for moving the rod 3 allows lifting the free end of the rod 3 with respect to the basement 2 and releasing it, subsequently, freely dropping against the test piece 7. Furthermore, this movement may be repeated cyclically.

The moving device 5, figures 1 and 2, comprises at least driving means 15, at least one transmission 16 and at least one rotating element 17 rotated by means of the driving means 15 and the transmission 16.

The driving means 15, for example a gearmotor unit with electric motor, are connected by means of a transmission 16 to the rotating element 17, thus allowing rotating the rotating element 17 around the axis 18 thereof.

The rotating element 17 - for example a cylindrical disc, as indicated in figures 1 and 2, or equivalent means, for example one or more radially arranged rotating arms (not illustrated) - is provided on one or on both sides of one or more pins 20 which allow a motion transmission from the rotating element 17 to the rod 3 according to the methods described in detail hereinafter.

Possible embodiments of the pins 20 may be, for example, made of pegs, stationary and/or able to rotate, constrained and/or removable from the rotating element 17.

The pins 20, may be obtained, for example, by means of wheels, allowing eliminating or reducing friction and dragging between the rod 3 and the pin 20 reducing the friction or contact wear of both components.

For such purpose, it is possible to reinforce the surface of the rod 3 which comes into contact with the pin 20 providing for the use of a special reinforcement and/or coating material, or a special surface treatment.

The connection of the pins 20 to the rotating element 17 may be obtained through different ways.

For example one or more pins 20 can be connected on only one side of the rotating element 17, by means of apposite blind and threaded seats 19 obtained on the rotating element 17, or one or more pairs of pins 20 can be connected on both sides of the rotating element 17 through special openings on the rotating element 17 to allow the mutual coupling of the pins 20.

The test material 7 is shaped, preferably but not exclusively, in a prismatic test piece so as to be housed on the support 6.

However samples of material to be tested of different shapes, for example a complete or partial footwear, soles, midsoles and insoles - elements being mainly subjected to several impacts during use - can be arranged on the support 6.

The support 6 is positioned on the basement 2 by means of a guide 23. Varying the relative positioning of the support 6 with respect to the guiding means 4, figures 1 and 2, allows modifying the impact parameters of the rod 3 against the test piece 7. Furthermore a more accurate positioning of the support 6 along the guide 23 can be obtained by means of a graduated reference scale 22 provided both on the rod 3 and on the basement 2, as indicated in figures 1 and 2.

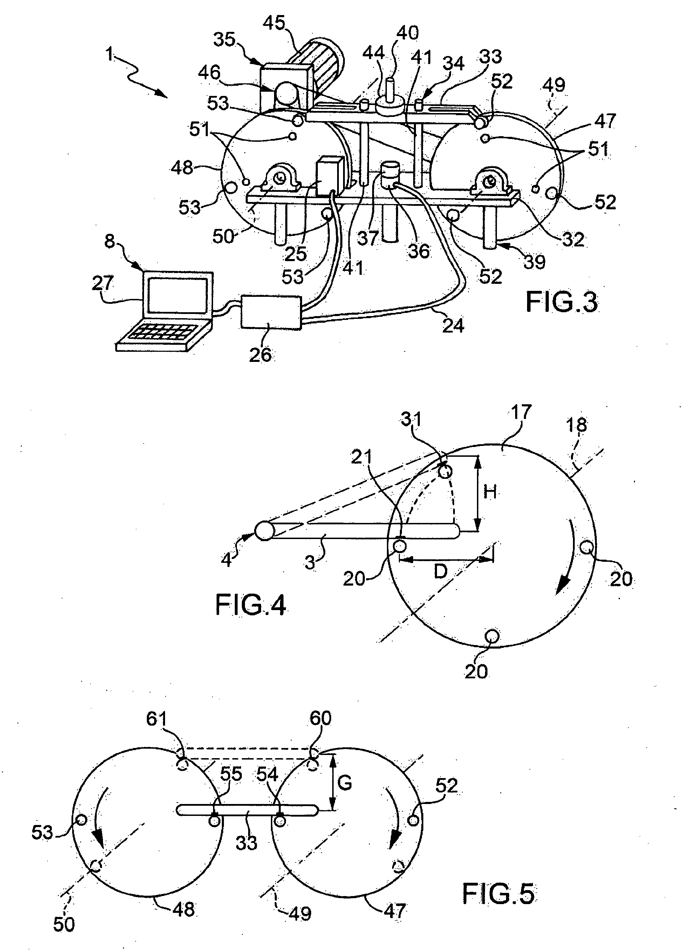

With reference to the embodiment of figure 3, the machine 1 mainly comprises a basement 32, a movable rod 33, means 34 for guiding the rod 33 with respect to the basement 32, a device 35 for moving the rod 33, a support 36 constrained to the basement 32, on which the material to be tested 37 is arranged, and an electronic device 8 for extrapolating the data regarding the stress and deformation to which the test material 37 is subjected.

The basement 32 is provided with a series of feet 39 for allowing the basement 32 to rest on the ground. The guiding means 34 may be obtained by means of a pair of tubular elements, for example with cylindrical section 41, as indicated in figure 3, or square-shaped section, blocked on the basement 32 and passing through suitable openings in the rod 33, or by means of a bead recirculation linear guide, not indicated in the figures, which is fastened to the basement 32 and constrained to the rod 33, allowing a relative translation of the rod 33 with respect to the basement 32 with a down-upward motion and vice versa.

The rod 33 is provided with a suitable support 40 for connecting and fixing a mass 44 to the rod 33. Modifying the mass 44 allows varying the force with which the rod 33 impacts against the test material 37, stressing the material with impacts of various degrees and simulating, for example, the stress to which, the materials used for making soles, midsoles and insoles are usually subjected to when walking or running.

The moving device 35 of the rod 33 allows lifting the rod 33 with respect to the basement 32 and releasing it, subsequently, freely dropping against the test piece 37. Furthermore, this movement may be repeated cyclically.

The moving device 35, comprises at least driving means 45, at least one transmission 46 and at least one pair of rotating elements 47, 48 rotated by means of the driving means 45 and the transmission 46.

The driving means 45, for example a gearmotor with electric motor, are connected by means of a transmission 46 to the pair of rotating elements 47 and 48, thus allowing rotating them in a synchronized manner around the respective axes 49 and 50.

The transmission 46 is also provided with suitable adjustment means, not indicated in the figure, for adjusting the synchronism of the rotation of the two rotating elements 47 and 48 and thus allowing a uniform lifting on the sides of the rod 33.

The rotating elements 47 and 48, for example a pair of discs, figure 3, or a pair of equivalent elements, are provided on one or both sides of one or more pins 52 which allow the transmission of motion from the rotating elements 47 and 48 to the rod 33.

Possible embodiments of the pins 52 may also in this case for example be pegs, stationary and/or able to rotate, constrained and/or removable from rotating elements 47 and 48.

The pins 52, may be obtained, for example, by means of wheels, allowing eliminating or reducing the friction and dragging between the rod 33 and the pin 52 and between the rod 33 and the pin 53, reducing the friction or contact wear of both components. Analogously to the previously illustrated version, the surface of the rod 33 which comes into contact with the pins 52 and 53 may be reinforced, providing for the use of a special reinforcement and/or coating material, or with a surface treatment.

The connection of the pins 52 to the rotating element 47 and of the pins 53 to the rotating element 48 could be obtained in different ways. For example one or more pins 52 can be connected on only one side of the rotating element 47, by means of special blind and threaded seats 51 obtained on the rotating element 47, or one or more pairs of pins 52 can be connected on both sides of the rotating element 47 through special openings on the rotating element 47 to allow the mutual coupling of the pins 52. Analogous considerations shall apply for the connection of the pins 52 to the rotating element 48. With reference to the embodiments of figures 1 and 3, moving the rod 3 or 33, with respect to the basement 2 or 32 occurs by rotating the circular element 17 or elements 47 and 48 around respective axes 18, 49, 50 following the actuation of the moving device 5 or 35. As indicated in figure 4, when rotating the rotating element 17, a first contact between the pin 20 and the rod 3 at the contact point 21 occurs. Subsequently the pin 20, travelling a circular trajectory, during the rotation of the rotating element 17, induces a progressive lift and rotation of the rod 3, constrained by means of the hinge 11 to the basement 2, up to reaching a maximum lift height H.

The continuous rotation of the rotating element 17 moves the position of the pin 20 beyond the point 31 of contact with the rod 3. Thus, the rod 3, no longer being supported by the pin 20, freely drops from up downwards due to the force of gravity against the test piece 7, as indicated in figure 1.

Analogously, as indicated in figure 5, during the rotation of the rotating elements 47 and 48, a first contact between the pin 52 and the rod 33 at the contact point 54 and between the pin 53 and the rod 33 at the contact point 55 is obtained.

Subsequently each pin 52 and 53, travels a circular trajectory, during the synchronous rotation of the rotating element 49 and 50, inducing the progressive translation of the rod 33 from down upwards, constrained by means of the guiding means 34 to the basement 32, up to reaching the maximum lift height G.

The continuous synchronous rotation of the rotating element 47 and 48 displaces, respectively, the pin position 52 beyond the point 60 of contact with the rod 33 and the pin position 53 beyond the point 61 of contact with the rod 33. Thus, the rod 33, no longer being supported by the pins 52 and 53, freely drops from up downwards due to the force of gravity against the test piece 37, as indicated in figure 3.

During the step of testing the material, data regarding the stress and deformation to which the test piece is subjected can be extrapolated by means of an electronic device 8, which comprises at least one strength electronic sensor 24, at least one position sensor 25, and a computer 27 or any other analogous data processing means.

The strength sensor 24 and the position sensor 25 are connected to an electronic signal acquisition card 26.

The electronic signal acquisition card 26, in turn, is connected to the computer 27, to allow receiving the signals sent from the sensors 24 and 25, and allow processing them by means of the computer 27 with the aim of defining the characteristics of the test material.

The strength electronic sensor 24, for example a strength transducer of the load cell type or other equivalent means, detects the intensity of the strength with which the rod 3 or 33, following the free drop due to the force of gravity, impacts the test piece.

The strength sensor 24 allows evaluating the elastic and damping properties of the test material, detecting the instantaneous variation curve of the load after each impact.

Furthermore, it also allows determining the deterioration of the mechanical properties of the material during the test.

A material which initially reveals to be extremely efficient at absorbing the impacts, may reveal an early deterioration of the mechanical properties following several stress cycles with respect to a material which, though initially revealing only a discrete absorption capacity, is however, more stable and efficient at bearing impacts during the fatigue test.

Preferably, the strength electronic sensor 24 is positioned between the support 6 or 36 and the test piece 7 or 37.

The position sensor 25, for example a laser pointer device or other equivalent means such as resistive sensors and/or an encoder, allows directly determining the relative position of the rod 3 or 33 with respect to the basement 2 or 32 during the test, and, indirectly, obtaining also the speed and the acceleration of the rod 3 or 33 during the test. Regarding the embodiment of figures 1 and 2 the position sensor 25 is positioned on the basement 2 near the hinge 11.

Furthermore, measuring the instantaneous position of the rod 3 or 33 by means of the position sensor 25 allows obtaining the deformation of the test piece 7 or 37 and the hysteresis of the material during the test.

The high sampling frequency of the position sensor signal 25, for example up to 1000 Hz, allows accurately determining the relative position of the rod 3 or 33 with respect to the test basement 2 or 32 during the test, and in particular during the step of impacting the rod 3 or 33 against the test piece 7 or 37, allowing characterizing the behaviour of the material.

The possible rebound of the rod 3 or 33 against the test piece 7 or 37 subsequent to the impact of the rod 3 or 33 against the test piece 7 or 37, indicates a poor capacity of the test material to absorb and damp the impacts.

The machine 1 may possibly comprise an acceleration sensor, for example an accelerometer, not indicated in the figure, positioned on the bar 3 or 33, for directly determining the dynamic parameters of the rod 3 or 33 and the test piece 7 or 37 during the test.

The operation of the present invention regarding the embodiment of figure 1 and 2 is as follows.

To carry out a fatigue test on a material characterised by a cyclic impact, the rod 3, figure 1 and 2, is placed at contact with the support means 10 to allow safely positioning the support 6 on the basement 2 and positioning the test material 7 on the support 6.

After positioning and blocking the material 7 on the support 6 and after positioning and blocking the support 6 on the basement 2 the rod 3 is placed at contact with the test piece 7.

Thus, the rotating element 17 is rotated by means of the driving means 15 and the transmission 16.

After the rotation of the rotating element 17 the contact of the pin 20 with the movable rod 3 at the contact point 21 is initially determined, figure 4, and, subsequently, the progressive lifting and rotation of the rod 3 constrained to the basement 2 by means of the hinge 11.

The rotation and lifting of the rod 3 with respect to the basement 2 continue during the rotation of the rotating element 17 up to when the pin 20 is at contact with the rod 3 at the contact point 31, reaching the maximum lift height H, figure 4. The rotation of the rotating element 17 moves the pin 20 away from the rod 3. The rod 3, not being supported by the pin 20, freely drops from up downwards due to the force of gravity up to impacting against the test material 7.

The lift and subsequent drop of the rod 3 against the material 7 are repeated cyclically due to the continuous rotation of the rotating element 17.

The force with which the rod 3 impacts the test piece 7, the position of the rod 3, and the acceleration with which the rod 3 is moved during the test are detected by means of the electronic signals sent from the strength sensor 24 and from the position sensor 25, to the computer 27 by means of a signal acquisition card 26. The signals are subsequently processed by means of the computer 27 extrapolating the stress/deformation constitutive bond curve of the tested material, during the test.

The operation of the present invention regarding the embodiment of figure 3 is analogous to what is described regarding figures 1 and 2 mainly except for the movement of the rod 33.

The rod 33 is constrained to the basement 32 by means of guiding means 41 which allow the rod 33 a translation relative to the basement 2 with a up-downward motion and vice versa.

The impact which is obtained between the rod 33 and the test piece 37 is without the transverse components which instead are present in the embodiment of figure 1 and 2 due to the relative rotation of the rod 3 with respect to the basement 2. The machine 1 according to the present invention is characterised by a great versatility.

Actually, it allows carrying out impact, and cyclic impact, tests allowing verifying and quantifying the resistance of the material to fatigue.

Furthermore, special electronic devices 8, allow extrapolating the stress-deformation constitutive bond curves of the test material and determining the deterioration of the properties of the material following several impacts.

The machine 1 allows varying, in a simple and practical manner, various parameters for performing tests, including the drop height of the rod 3 or 33 against the test material, the positioning of the test material 7 or 37 with respect to the rod 3 or 33, the frequency of the impacts of the rod 3 or 33 against the material 7 or 37, and the shape of the material 7 or 37 to be tested.

## Claims

1. Machine (1) to perform fatigue tests on a test material (7; 37), comprising at least a basement (2; 32), a movable rod (3; 33) having a free end, means (4; 34) for guiding the rod (3; 33) with respect to the basement (2; 32), a device (5; 35) for cyclic moving of the rod (3; 33), for the lifting of said free end of said rod (3; 33) with respect to said basement (2) and releasing it, subsequently, freely dropping it against said test material (7, 37), a support (6; 36) to position the test material (7; 37) on the basement (2; 32) and a test data acquisition electronic device (8), **characterised in that** said test data acquisition electronic device (8) comprises a position sensor (25), adapted to measure the instantaneous displacement of the rod (3; 33).

2. Machine according to claim 1, wherein said cyclic moving device (5; 35) comprises actuating means (15-17, 20; 45-48, 52) adapted to cyclically lifting said rod (3; 33).

3. Machine according to claim 1 or 2, wherein said cyclic moving device (5; 35) comprises at least a rotating element (17; 47, 48) provided with at least a pin (20; 52, 53) stationary and/or movable on one of and/or both sides adapted to lift said rod (3; 33).

4. Machine according to one or more of the previous claims, wherein said cyclic moving device (5; 35) comprises at least some driving means (15; 45) and at least a transmission (17; 37) to rotate said rotating element (17; 47, 48).

5. Machine according to claim 3 or 4, wherein said pin (20, 51) is contacted with said rod (3; 33).

6. Machine according to one or more of the previous claims, wherein said guiding means (4; 34) comprise a hinge (11) or one or more linear guides (41).

7. Machine according to one or more of the previous claims, wherein said rod (3; 33) comprises a support (13; 40) for positioning a mass (14; 44) which can be changed to increase or decrease the impact strength.

8. Machine according to one or more of the previous claims, wherein said electronic device (8) comprises a strength sensor (24) placed between the support (6; 36) and the test material (7; 37) suitable to measure the strength transmitted by said test material (7; 37).

9. Machine according to one or more of the previous claims, wherein said electronic device (8) comprises an electronic signal acquisition card (26) and at least a computer (27).

10. Machine according to claim 1 or 9, wherein said strength sensor (24) and said position sensor (25) are connected to said electronic signal acquisition card (26).

## Patentansprüche

1. Maschine (1) zum Ausführen von Ermüdungsversuchen an einem Prüfmaterial (7; 37), umfassend mindestens ein Untergestell (2; 32), einen beweglichen Stab (3; 33) mit einem freien Ende, Mittel (4; 34) zum Führen des Stabs (3; 33) in Bezug auf das Untergestell (2; 32), eine Vorrichtung (5; 35) zum zyklischen Bewegen des Stabs (3; 33), um das freie Endes des Stabs (3; 33) vom Untergestell (2) anzuheben und es anschließend wieder loszulassen und frei gegen das Prüfmaterial (7, 37) fallenzulassen, einen Träger (6; 36) zum Positionieren des Prüfmaterials (7; 37) auf dem Untergestell (2; 32) und eine elektronische Prüfdatenerfassungsvorrichtung (8), **dadurch gekennzeichnet, dass** diese elektronische Prüfdatenerfassungsvorrichtung (8) einen Positionssensor (25) umfasst, der geeignet ist, die momentane Auslenkung des Stabs (3; 33) zu messen.

2. Maschine nach Anspruch 1, wobei die Vorrichtung (5; 35) zum zyklischen Bewegen Betätigungsmittel (15-17, 20; 45-48, 52) umfasst, die geeignet sind, den Stab (3; 33) zyklisch anzuheben.

3. Maschine nach Anspruch 1 oder 2, wobei die Vorrichtung (5; 35) zum zyklischen Bewegen mindestens ein Drehelement (17; 47, 48) umfasst, das auf einer und/oder beiden Seiten mit mindestens einem ortsfesten und/oder beweglichen Zapfen (20; 52, 53) versehen ist, der geeignet ist, den Stab (3; 33) anzuheben.

4. Maschine nach einem oder mehreren der vorhergehenden Ansprüche, wobei die Vorrichtung (5; 35) zum zyklischen Bewegen mindestens einige Antriebsmittel (15; 45) und mindestens ein Getriebe (17; 37) zum Drehen des Drehelements (17; 47, 48) umfasst.

5. Maschine nach Anspruch 3 oder 4, wobei der Zapfen (20, 51) in Berührung mit dem Stab (3; 33) ist.

6. Maschine nach einem oder mehreren der vorhergehenden Ansprüche, wobei die Führungsmittel (4; 34) ein Gelenk (11) oder eine oder mehrere Linearführungen (41) umfassen.

7. Maschine nach einem oder mehreren der vorhergehenden Ansprüche, wobei der Stab (3; 33) einen Träger (13; 40) zum Anordnen einer Masse (14; 44) umfasst, die geändert werden kann, um die Aufprallstärke zu erhöhen oder zu verringern.

8. Maschine nach einem oder mehreren der vorhergehenden Ansprüche, wobei die elektronische Vorrichtung (8) einen zwischen dem Träger (6; 36) und dem Prüfmaterial (7; 37) angeordneten Kraftsensor (24) umfasst, der geeignet ist, die vom Prüfmaterial (7; 37) übertragene Kraft zu messen.

9. Maschine nach einem oder mehreren der vorhergehenden Ansprüche, wobei die elektronische Vorrichtung (8) eine elektronische Signalerfassungskarte (26) und mindestens einen Computer (27) umfasst.

10. Maschine nach Anspruch 1 oder 9, wobei der Kraftsensor (24) und der Positionssensor (25) mit der elektronischen Signalerfassungskarte (26) verbunden sind.

## Revendications

1. Machine (1) pour effectuer des essais de fatigue sur un matériau d'essai (7; 37), comprenant au moins un socle (2; 32), une tige mobile (3; 33) ayant une extrémité libre, des moyens (4; 34) pour guider la tige (3; 33) par rapport au socle (2; 32), un dispositif (5; 35) pour le déplacement cyclique de la tige (3; 33), pour le soulèvement de ladite extrémité libre de ladite tige (3; 33) par rapport audit socle (2) et son relâchement, successivement, en la laissant chuter librement contre ledit matériau d'essai (7; 37), un support (6; 36) pour positionner le matériau d'essai (7; 37) sur le socle (2; 32) et un dispositif électronique d'acquisition de données d'essai (8), **caractérisée en ce que** ledit dispositif électronique d'acquisition de données d'essai (8) comprend un capteur de position (25), adapté pour mesurer le déplacement instantané de la tige (3; 33).

2. Machine selon la revendication 1, dans laquelle ledit dispositif de déplacement cyclique (5; 35) comprend des moyens d'actionnement (15-17, 20; 45-48, 52) adaptés pour soulever de manière cyclique ladite tige (3; 33).

3. Machine selon la revendication 1 ou 2, dans laquelle ledit dispositif de déplacement cyclique (5; 35) comprend au moins un élément tournant (17; 47, 48) muni d'au moins une goupille (20; 52, 53) fixe et/ou mobile sur un et/ou les deux côtés, adaptée pour soulever ladite tige (3; 33).

4. Machine selon une ou plusieurs des revendications précédentes, dans laquelle ledit dispositif de déplacement cyclique (5; 35) comprend au moins certains moyens d'entraînement (15; 45) et au moins une transmission (17; 37) pour faire tourner ledit élément tournant (17; 47, 48).

5. Machine selon la revendication 3 ou 4, dans laquelle ladite goupille (20, 51) vient en contact avec ladite tige (3; 33).

6. Machine selon une ou plusieurs des revendications précédentes, dans laquelle lesdits moyens de guidage (4; 34) comprennent une articulation (11) ou un ou plusieurs guides linéaires (41).

7. Machine selon une ou plusieurs des revendications précédentes, dans laquelle ladite tige (3; 33) comprend un support (13; 40) pour positionner une masse (14; 44) qui peut être changée pour augmenter ou diminuer la force d'impact.

8. Machine selon une ou plusieurs des revendications précédentes, dans laquelle ledit dispositif électronique (8) comprend un capteur de force (24) placé entre le support (6; 36) et le matériau d'essai (7; 37) adapté pour mesurer la force transmise par ledit matériau d'essai (7; 37).

9. Machine selon une ou plusieurs des revendications précédentes, dans laquelle ledit dispositif électronique (8) comprend une carte d'acquisition de signal électronique (26) et au moins un ordinateur (27).

10. Machine selon la revendication 1 ou 9, dans laquelle ledit capteur de force (24) et ledit capteur de position (25) sont connectés à ladite carte d'acquisition de signal électronique (26).
